# EUROPÄISCHE PATENTSCHRIFT

(11) **EP 0 995 411 B1**
(45) Veröffentlichungstag und Bekanntmachung des Hinweises auf die Patenterteilung: **06.06.2007**
(21) Anmeldenummer: 99120067.6
(22) Anmeldetag: 19.10.1999
(51) Int. Cl.: A61F 2/36, A61F 2/46, A61F 2/30

(54) **Zementierbare Endoprothese**
Cementable endoprosthesis
Endoprothèse cimentable

(30) Priorität: 21.10.1998 DE 19848476
(43) Veröffentlichungstag der Anmeldung: 26.04.2000
(73) Patentinhaber: Aesculap AG & Co. KG, 78532 Tuttlingen (DE)
(72) Erfinder: Grupp, Thomas, Dipl.-Ing., 73072 Donzdorf (DE); Pfaff, Andreas, 78532 Tuttlingen (DE); Hermle, Thomas, Dipl.-Ing, 78628 Rottweil (DE); Strohm Jürgen, Dipl.-Ing, 78056 VS-Schwenningen (DE)
(74) Vertreter: Hoeger, Stellrecht & Partner Patentanwälte

(56) Entgegenhaltungen:
- EP-A- 0 428 127
- WO-A-94/07439
- DE-A- 19 613 081
- US-A- 4 274 163
- US-A- 5 693 099

## Beschreibung

Die Erfindung betrifft eine Endoprothese mit einem in einen Knochenhohlraum einführbaren Schaft, der eine sich an seiner Außenseite öffnende Knochenzementzufuhrleitung aufnimmt und der im eingeführten Zustand mittels eines Dichtelementes den Knochenhohlraum proximal verschließt.

Schaftförmige Endoprothesen können zementlos und unter Verwendung von aushärtbarem Knochenzement in einem Knochenhohlraum befestigt werden. Bei Verwendung des Knochenzementes werden die Schaftendoprothesen in den Knochenhohlraum eingesetzt und der Zwischenraum zwischen Schaft und der Wand des Knochenhohlraumes wird mit einem Knochenzement ausgefüllt, der nach dem Einbringen aushärtet.

Es ist bekannt, den Knochenzement über eine Knochenzementzufuhrleitung im Inneren des Schaftes in den Knochenhohlraum einzubringen, beispielsweise beschreibt die EP 0 428 127 B1 eine solche Schaftendoprothese, bei der Knochenzement über die gesamte Länge des Schaftes in den Knochenhohlraum eingefüllt werden kann.

Bei anderen Schaftendoprothesen erfolgt die Einfüllung des Knochenzementes am distalen Ende des Schaftes, so daß der Knochenhohlraum vom distalen zum proximalen Ende des Schaftes kontinuierlich mit Knochenzement aufgefüllt wird.

Eine Endoprothese gemäß der Präambel des Anspruchs 1 wird in DE 196 13 081 beschrieben.

Es hat sich herausgestellt, daß es sehr schwer ist, die Druckverhältnisse im Knochenhohlraum beim Einfüllen des Knochenzementes zu kontrollieren, zumal da sich die Viskosität des eingefüllten Knochenzementes zeitlich ändert, so daß davon auszugehen ist, daß Knochenzement mit unterschiedlicher Viskosität eingefüllt wird. Um eine homogene Befüllung des Knochenhohlraumes zu erreichen, ist es wesentlich, daß diese Druckverhältnisse genau reproduzierbar sind, außerdem müssen zu hohe Druckwerte in jedem Fall vermieden werden, da durch zu hohe Druckwerte Verletzungen der Knochensubstanz erfolgen können.

Es ist Aufgabe der Erfindung, eine gattungsgemäße Endoprothese so auszubilden, daß sie auch bei unterschiedlicher Viskosität des eingefüllten Knochenzementes die Befüllung des Knochenhohlraumes unter kontrollierbaren Druckverhältnissen ermöglicht.

Diese Aufgabe wird bei einer Endoprothese der eingangs beschriebenen Art erfindungsgemäß dadurch gelöst, daß die Knochenzementzufuhrleitung ausschließlich in einem sich direkt an das Dichtelement anschließenden oberen Abschnitt des Schaftes in den Knochenhohlraum einmündet und daß in dem Dichtelement mindestens eine den Knochenhohlraum mit der Umgebung verbindende Belüftungsöffnung angeordnet ist.

Bei einer solchen Ausgestaltung erfolgt die Befüllung des Knochenhohlraumes an dessen Oberseite, also im proximalen Teil der Schaftendoprothese, und der in diesem Bereich in den Knochenhohlraum eintretende Knochenzement fließt von der Eintrittsstelle sowohl in distaler Richtung als auch in proximaler Richtung, es erfolgt also eine Aufteilung des Knochenzementstromes. Der in proximaler Richtung fließende Strom wird sehr schnell durch das proximale Dichtelement begrenzt, in proximaler Richtung erfolgt dann lediglich ein Abströmen des Knochenzementes durch die Belüftungsöffnung des Dichtelementes, in distaler Richtung hingegen kann der Knochenzement frei in den Knochenhohlraum eintreten.

Durch das Abströmen des Knochenzementes durch die Belüftungsöffnung erfolgt eine Reduzierung des Druckes im zugeführten Knochenzement, und zwar entsprechend den Querschnittsverhältnissen der Belüftungsöffnung in genau definierter Weise.

Es hat sich nun überraschenderweise herausgestellt, daß bei einer solchen Ausgestaltung der Endoprothese der sich im Knochenhohlraum maximal aufbauende Druck im wesentlichen unabhängig ist von der Viskosität des Knochenzementes, so daß vollständig reproduzierbare und im wesentlichen durch den Querschnitt der Belüftungsöffnung bestimmte Druckverhältnisse im Knochenhohlraum erzielt werden können. Dies führt zu einer optimalen Qualität des Knochenzementmantels, und es hat sich auch herausgestellt, daß dieser Knochenzementmantel äußerst homogen aufgebaut ist.

Der Knochenzement füllt den Knochenhohlraum bei der beschriebenen Endoprothese von proximal nach distal. Der Füllvorgang kann abgebrochen werden, wenn eine bestimmte Einfülltiefe erreicht ist, so daß auf diese Weise eine Hybridzementierung erreicht werden kann, bei der der Schaft im distalen Bereich nicht von Knochenzement umgeben ist. Es ist aber auch möglich, den Knochenhohlraum vollständig mit Knochenzement auszufüllen, sobald die Füllung vollständig ist, tritt weiter zugeführter Knochenzement quantitativ durch die Belüftungsöffnung aus und verhindert so einen übermäßigen Druckanstieg im Inneren des Knochenhohlraums.

Das Dichtelement kann ein mit dem Schaft einstückig verbundener Kragen sein, der vorzugsweise in den Knochenhohlraum dichtend eingesteckt ist, der aber auch zusätzlich oder statt dessen auf einer Knochenschnittfläche am proximalen Ende des Knochenhohlraumes aufliegen kann.

Bei einer anderen Ausführungsform ist vorgesehen, daß das Dichtelement ein den Schaft ringförmig umgebendes, separates Bauteil ist. Dieses kann beispielsweise ringförmig oder mehrteilig ausgebildet sein und mit dem Schaft durch einen Klemmsitz oder durch eine Verklebung verbunden sein.

Insbesondere kann das Dichtelement aus Knochenzement bestehen, so daß nach Befüllung des Knochenhohlraumes mit Knochenzement sich der Knochenzement des Dichtelementes und der eingefüllte Knochenzement zu einem einheitlichen Knochenzementmantel verbinden.

Der Querschnitt der Belüftungsöffnung oder gegebenenfalls der Belüftungsöffnungen insgesamt kann zwischen 1 mm² und 150 mm² liegen, vorzugsweise zwischen 5 mm² und 15 mm².

Bei einer bevorzugten Ausführungsform ist vorgesehen, daß der Schaft am distalen Ende ein Zentrierelement trägt, so daß er vor dem Einfüllen des Knochenzementes in genau definierter Zentrierlage im Knochenhohlraum festgelegt werden kann.

Weiterhin ist bei einer bevorzugten Ausführungsform möglich, daß der Schaft am distalen Ende ein Abdichtelement trägt, durch welches der Knochenhohlraum abgedichtet wird. Dadurch wird bei einer vollständigen Befüllung des Knochenhohlraumes sichergestellt, daß der Knochenzement nur eine begrenzte Eindringtiefe erhält und nicht wesentlich über die Länge des Schaftes der Endoprothese hinaus in den Knochenhohlraum eindringt.

Gegebenenfalls können Zentrierelement und Abdichtelement durch ein einziges Bauteil realisiert werden.

Der Schaft kann mit dem Zentrierelement und/oder dem Abdichtelement über einen Adapter verbunden sein, so daß Zentrierelement und/oder Abdichtelement an den Schaft angesetzt werden können, beispielsweise durch Einstecken in eine vorgesehene Öffnung.

Insbesondere ist es vorteilhaft, wenn der Adapter aus Knochenzement besteht. Bei einer vollständigen Befüllung des Knochenhohlraumes mit Knochenzement verbindet sich somit der eingefüllte Knochenzement mit dem Knochenzement des Adapters zu einem einheitlichen Knochenzementmantel.

Zur Lösung der oben beschriebenen Aufgabenstellung besitzt der Schaft weiterhin eine Entlüftungsöffnung, die im Bereich des distalen Endes des Schaftes in den Knochenhohlraum austritt. Durch diese Entlüftungsleitung, die einfach in die Umgebung geöffnet sein kann, die aber auch mit einer Saugquelle verbunden sein kann, werden beim Befüllen des Knochenhohlraumes mit Knochenzement Körperflüssigkeiten und Gase abgesaugt, nach vollständiger Befüllung des Knochenhohlraumes tritt durch diese Entlüftungsleitung dann auch Knochenzement aus und zeigt das Ende der Befüllung an. Die Entlüftungsleitung dient während des Befüllungsvorganges außerdem der Vermeidung von Druckspitzen im Knochenhohlraum.

Die nachfolgende Beschreibung dient im Zusammenhang mit den Zeichnungen der näheren Erläuterung. Es zeigen:
- Figur 1:: eine Längsschnittansicht einer in einen Knochenhohlraum eingesetzten Schaftendoprothese mit angeformtem Dichtkragen und
- Figur 2:: eine Ansicht ähnlich Figur 1 einer Schaftendoprothese mit einem separaten Dichtelement am proximalen Ende und mit einem Markraumstopper am distalen Ende.

Die Endoprothese der Erfindung ist für das Einsetzen in verschiedenartige Röhrenknochen geeignet, beispielsweise in den Femur, sie kann jedoch auch im Bereich des Beckens oder der Schulter eingesetzt werden. Nachstehend wird die Erfindung an einer Schaftendoprothese erläutert, die in einen Femur 1 eingesetzt ist. Diese Schaftendoprothese umfaßt einen stielförmigen Schaft 2, dessen Querschnitt in distaler Richtung, also zu seinem unteren Ende hin, kontinuierlich abnimmt und der an seinem proximalen Ende ein Anschlußteil 3 für ein Gelenkelement aufweist, beispielsweise eine Gelenkkugel, die auf einen konusförmigen Abschnitt 4 des Anschlußteiles 3 aufgesetzt werden kann (in der Zeichnung nicht dargestellt).

Am proximalen Ende des Schaftes 2 an dessen Übergangsbereich zum Anschlußteil 3 trägt der Schaft 2 einen radial nach außen abstehenden Dichtkragen 5, der beim Einführen des Schaftes 2 in einen entsprechend vorbereiteten Knochenhohlraum 6 des Femurs 1 in diesen eintritt und diesen an seinem proximalen Ende verschließt. Der Dichtkragen 5 könnte auch radial abstehende, flanschartige Vorsprünge tragen, die sich auf die Schnittfläche des Femurs 1 auflegen, dies ist beim dargestellten Ausführungsbeispiel nicht gezeigt.

Im Dichtkragen 5 sind im dargestellten Ausführungsbeispiel zwei durchgehende Belüftungskanäle 7 angeordnet, die den Knochenhohlraum 6 mit der Umgebung verbinden. Diese Belüftungskanäle 7 haben einen relativ kleinen Querschnitt, er kann zwischen 1 mm² und 150 mm² insgesamt liegen, vorzugsweise zwischen 5 mm² und 15 mm².

Im proximalen Abschnitt des Schaftes 2 ist eine Knochenzementzufuhrleitung 8 aufgenommen, die geringfügig unterhalb des Dichtkragens 5 über eine seitliche Öffnung 9 in den Knochenhohlraum 6 einmündet. Die Einmündungsstelle muß nicht unmittelbar neben dem Dichtkragen 5 liegen, sondern sie kann in distaler Richtung geringfügig vom Dichtkragen 5 beabstandet sein, auf jeden Fall befindet sich aber diese seitliche Öffnung 9 im proximalen Abschnitt des Schaftes 2 in einem Abstand vom Dichtkragen, der maximal 40 %, vorzugsweise 20 % der Gesamtlänge des Schaftes entspricht.

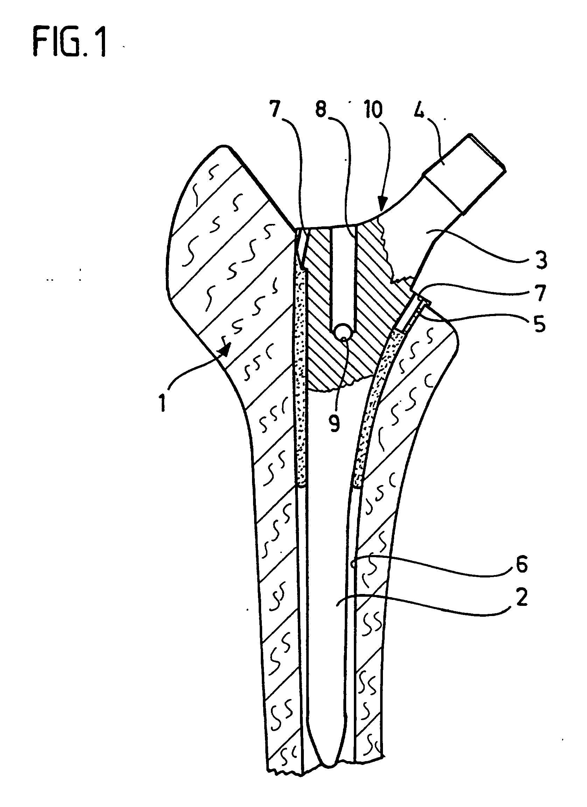

Zur Zementierung der in Figur 1 dargestellten Endoprothese 10 wird der Schaft 2 in den entsprechend vorbereiteten Knochenhohlraum 6 eingeführt und in diesem in der gewünschten Einbaulage positioniert, wobei der Knochenhohlraum 6 in der beschriebenen Weise durch den Dichtkragen 5 abgeschlossen wird.

Der Schaft 2 kann ein Zentrierelement tragen, das in der Darstellung der Figur 1 nicht gezeigt ist, und das sicherstellt, daß der Schaft 2 im Inneren des Knochenhohlraumes 6 allseits den gewünschten Abstand von der Wand des Knochenhohlraumes 6 einhält.

Nach dieser Positionierung des Schaftes 2 im Knochenhohlraum 6 wird an die Knochenzementzufuhrleitung 8 eine Zufuhrleitung für Knochenzement angeschlossen, diese kann beispielsweise mit einem herkömmlichen Gerät zur Bereitstellung von fließfähigem Knochenzement verbunden werden. Der Knochenzement, der normalerweise kurz vor der Applikation zubereitet wird und von dem Augenblick der Zubereitung an eine zunehmende Viskosität aufweist, wird in einem Zeitpunkt eingebracht, in dem diese Viskosität in einem gewünschten Bereich liegt, dies kann durch geeignete Maßnahmen überprüft werden.

Die Zufuhr erfolgt über die Quelle vorzugsweise mit einem konstanten Volumenstrom, und auf diese Weise tritt der viskose Knochenzement durch diese seitliche Öffnung 9 in den Knochenhohlraum 6 ein. Der eintretende Strom des Knochenzementes teilt sich im Knochenhohlraum in zwei Teilströme auf, nämlich einen ersten Teilstrom, der in proximaler Richtung gerichtet ist, und einen zweiten Teilstrom, der in distaler Richtung gerichtet ist. Der erste Teilstrom füllt nach kurzer Zeit den gesamten oberhalb der seitlichen Öffnung 9 verbleibenden und durch den Dichtkragen 5 begrenzten Teil des Knochenhohlraumes 6 aus, anschließend tritt der Knochenzement dieses Teilstromes durch die Belüftungskanäle 7 in die Umgebung aus. Diese Belüftungskanäle wirken als Drossel und verhindern somit, daß eine zu große Menge des Knochenzementes über diese Öffnungen austritt. Dadurch ist sichergestellt, daß der wesentliche Teil des eintretenden Knochenzementes nach der vollständigen Ausfüllung des oberen Teils des Knochenhohlraumes 6 in distale Richtung in den Knochenhohlraum 6 eintritt und diesen von oben nach unten sukzessive auffüllt.

Dieser Auffüllvorgang kann beendet werden, bevor der Knochenhohlraum 6 vollständig ausgefüllt ist, eine solche Teilfüllung oder Hybridfüllung ist im Ausführungsbeispiel der Figur 1 dargestellt.

Es ist aber auch möglich, den Knochenhohlraum 6 vollständig auszufüllen.

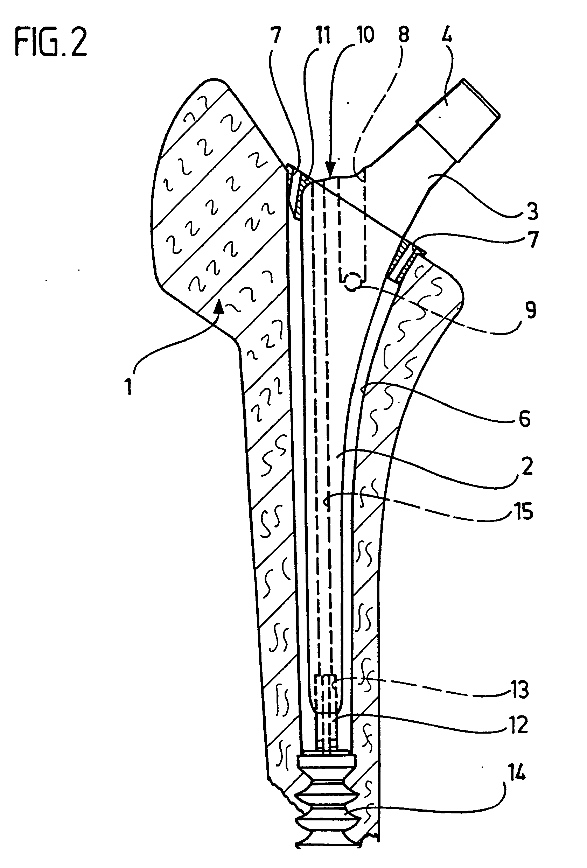

Die Endoprothese 10 gemäß Figur 2 ist ähnlich aufgebaut, einander entsprechende Teile tragen daher dieselben Bezugszeichen.

Im Unterschied zur Endoprothese 10 der Figur 1 trägt diese Endoprothese 10 keinen angeformten Dichtkragen, sondern der Schaft 2 ist an seinem proximalen Ende von einem ringförmigen Dichtelement 11 umgeben, welches zwischen Schaft 2 und der Innenwand des Knochenhohlraumes 6 in den Knochenhohlraum 6 einschiebbar ist und diesen dadurch nach außen hin abdichtet. Das Dichtelement 11 kann als auf den Schaft 2 aufgeschobener Ring ausgebildet sein, in anderen Fällen ist das Dichtelement 11 mehrteilig ausgebildet und die Teile sind von außen her am Schaft 2 festgelegt, beispielsweise durch Verklebung.

Insbesondere kann vorgesehen sein, daß das Dichtelement 11 aus ausgehärtetem Knochenzement besteht.

Am distalen Ende des Schaftes 2 ist dieser mit einem Adapter 12 verbunden, der beispielsweise in eine Einstecköffnung 13 am Schaftende 2 eingesteckt sein kann. Der Adapter 12 trägt einen sogenannten Markraumstopper 14, dies ist ein verformbarer Stopfen, der den Knochenhohlraum 6 nach unten hin abdichtet und der gleichzeitig den Schaft 2 im Knochenhohlraum 6 zentriert. Der Adapter 12 kann insbesondere aus bereits ausgehärtetem Knochenzement bestehen, jedoch sind auch andere körperverträgliche Materialien möglich.

Im Inneren des Schaftes 2 verläuft von dessen proximalem Ende ausgehend bis zu seinem distalen Ende eine Belüftungsleitung 15 entsprechend dem kennzeichnenden Teil des Anspruchs 1, die sich am distalen Ende des Schaftes 2 oder auch erst im Bereich des Adapters 12 und/oder des Markraumstoppers 14 in den Knochenhohlraum 6 öffnet und die somit eine Verbindung des distalen Endbereiches des Knochenhohlraumes 6 mit der Umgebung ermöglicht. Die Belüftungsleitung 15 kann am proximalen Ende des Schaftes 2 einfach in die Umgebung austreten, es kann dort aber auch eine Saugleitung einer Saugquelle angeschlossen werden, so daß über die Belüftungsleitung 15 das distale Ende des Knochenhohlraumes 6 mit einer Saugquelle verbunden wird.

Das Einsetzen des Schaftes 2 in den Knochenhohlraum 6 und die Befüllung mit Knochenzement erfolgt genau in der gleichen Weise wie beim Ausführungsbeispiel der Figur 1, jedoch wird hier während der Befüllung gegebenenfalls zusätzlich über die Belüftungsleitung 15 eine aktive oder passive Belüftung des Knochenhohlraumes 6 durchgeführt, so daß dadurch die Befüllungsqualität zusätzlich verbessert werden kann. Wenn der Dichtkragen 5 und/oder der Adapter 12 aus verfestigtem Knochenzement bestehen, kann sich der über die Knochenzementzufuhrleitung 8 eingefüllte Knochenzement nach dessen Aushärtung mit diesen Teilen einstückig verbinden, so daß eine vollständig homogene Ummantelung des Schaftes mit Knochenzement erreicht werden kann.

Im übrigen könnte selbstverständlich auch die Endoprothese 10 gemäß Figur 1 mit einem Markraumstopper und/oder einer Belüftungsleitung versehen werden, wie sie am Ausführungsbeispiel der Figur 2 dargestellt und erläutert worden sind.

## Patentansprüche

1. Endoprothese mit einem in einen Knochenhohlraum einführbaren Schaft, der eine sich an seiner Aussenseite öffnende Knochenzementzufuhrleitung aufnimmt und der im eingeführten Zustand mittels eines Dichtelementes den Knochenhohlraum proximal verschließt wobei in dem Dichtelement (5; 11) mindestens eine den Knochenhohlraum (6) mit der Umgebung verbindende Belüftungsöffnung (7) angeordnet ist, **dadurch gekennzeichnet, daß** die Knochenzementzufuhrleitung (8) nur in einem sich direkt an das Dichtelement (5; 11) anschließenden oberen Abschnitt des Schaftes (2) in den Knochenhohlraum (6) einmündet, und daß der Schaft (2) eine Entlüftungsleitung (15) aufnimmt, die im Bereich des distalen Endes des Schaftes (2) in den Knochenhohlraum (6) austritt.

2. Endoprothese nach Anspruch 1, **dadurch gekennzeichnet, daß** das Dichtelement (5) ein mit dem Schaft (2) einstückig verbundener Kragen ist.

3. Endoprothese nach Anspruch 1, **dadurch gekennzeichnet, daß** das Dichtelement (11) ein den Schaft (2) ringförmig umgebendes, separates Bauteil ist.

4. Endoprothese nach Anspruch 3, **dadurch gekennzeichnet, daß** das Dichtelement (11) aus Knochenzement besteht.

5. Endoprothese nach einem der voranstehenden Ansprüche, **dadurch gekennzeichnet, daß** der Querschnitt der Belüftungsöffnung (7) oder der Belüftungsöffnungen (7) insgesamt zwischen 1 mm² und 150 mm² liegt.

6. Endoprothese nach Anspruch 5, **dadurch gekennzeichnet, daß** der Querschnitt der Belüftungsöffnung (7) oder der Belüftungsöffnungen (7) insgesamt zwischen 5 mm² und 15 mm² liegt.

7. Endoprothese nach einem der voranstehenden Ansprüche, **dadurch gekennzeichnet, daß** der Schaft (2) am distalen Ende ein Zentrierelement (14) trägt.

8. Endoprothese nach einem der voranstehenden Ansprüche, **dadurch gekennzeichnet, daß** der Schaft (2) am distalen Ende ein Abdichtelement (14) trägt.

9. Endoprothese nach einem der Ansprüche 7 oder 8, **dadurch gekennzeichnet, daß** der Schaft (2) mit dem Zentrierelement (14) und/oder dem Abdichtelement (14) über einen Adapter (12) verbunden ist.

10. Endoprothese nach Anspruch 9, **dadurch gekennzeichnet, daß** der Adapter (12) aus Knochenzement besteht.

## Claims

1. Endoprosthesis having a shaft which is insertable into a bone cavity, accommodates a bone cement supply conduit, opening at the outside of the shaft, and in the inserted state proximally seals the bone cavity by means of a sealing element, at least one ventilation opening (7), which connects the bone cavity (6) to the surroundings, being arranged in the sealing element (5; 11), **characterised in that** the bone cement supply conduit (8) opens into the bone cavity (6) only in an upper section of the shaft (2) directly adjoining the sealing element (5; 11), and **in that** the shaft (2) accommodates a venting conduit (15) which emerges into the bone cavity (6) in the region of the distal end of the shaft (2).

2. Endoprosthesis according to Claim 1, **characterised in that** the sealing element (5) is a collar connected in one piece with the shaft (2).

3. Endoprosthesis according to Claim 1, **characterised in that** the sealing element (11) is a separate component annularly surrounding the shaft (2).

4. Endoprosthesis according to Claim 3, **characterised in that** the sealing element (11) is composed of bone cement.

5. Endoprosthesis according to one of the preceding claims, **characterised in that** the cross-section of the ventilation opening (7) or of the ventilation openings (7) as a whole is between 1 mm² and 150 mm².

6. Endoprosthesis according to Claim 5, **characterised in that** the cross-section of the ventilation opening (7) or of the ventilation openings (7) as a whole is between 5 mm² and 15 mm².

7. Endoprosthesis according to one of the preceding claims, **characterised in that** the shaft (2) carries a centring element (14) at the distal end.

8. Endoprosthesis according to one of the preceding claims, **characterised in that** the shaft (2) carries a sealing element (14) at the distal end.

9. Endoprosthesis according to one of Claims 7 and 8, **characterised in that** the shaft (2) is connected to the centring element (14) and/or the sealing element (14) via an adapter (12).

10. Endoprosthesis according to Claim 9, **characterised in that** the adapter (12) is composed of bone cement.

## Revendications

1. Endoprothèse comportant un fût qui peut être introduit dans une cavité osseuse, qui reçoit une conduite d'amenée de ciment osseux s'ouvrant sur sa face extérieure et qui, à l'état introduit, ferme la cavité osseuse côté proximal au moyen d'un élément d'étanchéité, au moins une orifice d'aérage (7) qui relie la cavité osseuse (6) avec l'environnement étant agencé dans l'élément d'étanchéité (5; 11), **caractérisée en ce que** la conduite d'amenée de ciment osseux (8) ne débouche dans la cavité osseuse (6) que dans un tronçon supérieur du fût (2), qui se raccorde directement à l'élément d'étanchéité (5 ; 11), et **en ce que** le fût (2) reçoit une conduite de désaération (15) qui sort dans la cavité osseuse (6), dans la région de l'extrémité distale du fût (2).

2. Endoprothèse selon la revendication 1, **caractérisée en ce que** l'élément d'étanchéité (5) est un col relié d'un seul tenant avec le fût (2).

3. Endoprothèse selon la revendication 1, **caractérisée en ce que** l'élément d'étanchéité (11) est un composant séparé entourant le fût (2) à la manière d'une bague.

4. Endoprothèse selon la revendication 3, **caractérisée en ce que** l'élément d'étanchéité (11) est en ciment osseux.

5. Endoprothèse selon l'une des revendications précédentes, **caractérisée en ce que** la section transversale de l'orifice ou des orifices d'aérage (7) est située dans l'ensemble entre 1 mm² et 150 mm².

6. Endoprothèse selon la revendication 5, **caractérisée en ce que** la section transversale de l'orifice ou des orifices d'aérage (7) est située dans l'ensemble entre 5 mm² et 15 mm².

7. Endoprothèse selon l'une des revendications précédentes, **caractérisée en ce que** le fût (2) porte un élément de centrage (14) à l'extrémité distale.

8. Endoprothèse selon l'une des revendications précédentes, **caractérisée en ce que** le fût (2) porte un élément d'étanchement (14) à l'extrémité distale.

9. Endoprothèse selon l'une des revendications 7 ou 8, **caractérisée en ce que** le fût (2) est relié à l'élément de centrage (14) et/ou à l'élément d'étanchement (14) via un adaptateur (12).

10. Endoprothèse selon la revendication 9, **caractérisée en ce que** l'adaptateur (12) est en ciment osseux.
